# EUROPEAN PATENT SPECIFICATION

(11) **EP 1 192 117 B1**
(45) Date of publication and mention of the grant of the patent: **01.09.2004**
(21) Application number: 00936602.2
(22) Date of filing: 15.06.2000
(51) Int. Cl.: C07C 45/29, C07C 47/21, A01N 35/02

(54) **(E8,Z10)-TETRADECA-8,10-DIENAL, THE METHOD OF ITS PREPARATION AND ITS USE AS SEXUAL ATTRACTANT FOR LEAFMINER MOTHS**
(E8,Z10)-TETRADECA-8,10-DIENAL,VERFAHREN ZU SEINER HERSTELLUNG UND SEINE VERWENDUNG ALS SEXUALLOCKSTOFF FÜR MINIERMOTTEN
(E8,Z10)-TETRADECA-8,10-DIENAL, PROCEDE DE SA PREPARATION ET SON UTILISATION EN TANT QUE SUBSTANCE D'ATTRACTION SEXUELLE POUR LES TEIGNES MINEUSES DES FEUILLES

(30) Priority: 16.06.1999 CZ 215699
(43) Date of publication of application: 03.04.2002
(73) Proprietor: Ustav Organicke Chemie A Biochemie Akademie Ved Ceske Republiky, 166 10 Praha 6 (CZ)
(72) Inventor: SVATOS, Ales,, 130 00 Praha 3 (CZ); KALINOVA, Blanka;, 181 00 Praha 8 (CZ); HOSKOVEC, Michal;, 120 00 Praha 2 (CZ); KINDL, Jiri,, 181 00 Praha 8 (CZ); HRDY, Ivan, 156 00 Praha 5 (CZ)
(74) Representative: Beetz & Partner Patentanwälte
(86) International application number: PCT/CZ2000/000044
(87) International publication number: WO 2001/000553

(56) References cited:
- US-A- 4 228 093
- SVATO, ALES ET AL: "Identification of a new lepidopteran sex pheromone in picogram quantities using an antennal biodetector: (8E,10Z)-tetradeca-8,10- dienal from Cameraria ohridella" TETRAHEDRON LETT. (1999), 40(38), 7011-7014 , XP002152306
- HOSKOVEC, MICHAL ET AL: "Synthesis of (8E,10Z)-tetradeca-8,10-dienal, sex pheromone of horse chestnut leafminer (Cameraria ohridella), and all its geometrical isomers" COLLECT. CZECH. CHEM. COMMUN. (2000), 65(4), 511-523 , XP002152307
- ANDO, TETSU ET AL: "Lepidopterous sex attractants with a conjugated diene system" AGRIC. BIOL. CHEM. (1987), 51(10), 2691-4 , XP000961546
- DESCOINS, C. ET AL: "Stereoselective syntheses of (E)-7,(Z)-9- dodecadien -1-yl acetate" SYNTH. COMMUN. (1984), 14(8), 761-73 , XP000961530
- BESTMANN, HANS JUERGEN ET AL: "Pheromone. XXXIV. Synthese konjugiert-ungesättigter Lepidopterenpheromone und Analoga" LIEBIGS ANN. CHEM. (1981), (12), 2117-38 , XP002152308

## Description

### Technical Field

This invention relates to moth attractants and to methods of synthesizing and using these attractants.

### Background Art

The horse chestnut leafminer is a new insect pest introduced to Europe around 1985. This tiny moth is a significant pest for the horse chestnut (*Aesculus hippocastanum* L.), on which the moth's larvae consume the leaf parenchyma during their development stage and form mines. When a massive infestation takes place, frequently during the second pest generation (July), the larvae cause necrosis and then shedding of the leaves. Due to the shortened assimilation period, the trees are weakened and where heavy infestation occurs continuously over several years, the trees can die. A more serious situation can come about when the leafminer larvae shift to another tree host, as we can currently witness on maple. In regions where leafminer infestation has occurred for ca 10 years, the parasitation of leafminer larvae by parasitoids from the genus Chalcidoidea has only reached 30%, an insufficient rate for effective control of the pest populations. The only suggested and useable methods of controlling the pest are the racking and burning of infested horse chestnut leaves [Skuhravy V. *Anzeiger für Schädlingskunde Pflanzenschutz Umweltschutz* **71**, 82-84 (1998)] and the spraying of trees with inhibitors of chitin biosynthesis (e.g. diflubenzuron).

Chemical communication serves diverse behavioral and physiological functions in nature. For example, chemicals named "sex pheromones" are used by individuals belonging to same species to find a partner of the opposite sex for mating. Those compounds can be chemically characterized and prepared in laboratory. So far we know the chemical structures of ca 400 sex pheromones of moths [Am, H., Tóth, M. M. 1998: List of Sex Pheromones of Lepidoptera and Related Attractants. In Am H., Tóth M. & Priesner E. (eds): *The Pherolist.* Internet database (www-pherolist.slu.se)]. At present no pheromone-based methods to protect the horse chestnut trees against the leafininer exist, however, those pheromone-based techniques of protection against other types of moth (the tortrix and pyralid moths) are much more advanced and are successfully used for population monitoring [Ridgeway, R.L., Silverstein, R.M. a May, N.I (Ed.) 1990: *Behavior-Modifying Chemicals for Insect Management.* Marcel Dekker, Inc. New York, 761 pp.], or to confuse the males, by making it more difficult for males to locate females for mating by increasing the airborne concentrations of synthetic pheromones (Ridgeway et al. 1990). Beside these simple techniques, some more complex ones have been developed, e.g. where males are lured by a synthetic pheromone in proximity to toxic fumigants and/or insecticides (the attract and kill method) (Hofer, D. and Brassel, J, "Attract and kill" to control *Cydia pomonella* and *Pectiophora gossypiella IOBC*/*WPRS Bull.* 1992/XV/5: 36-39), or to viral agents (Czech patent application PV 1680-95), or spores of entomofagous fungus (PV 1680-95) or compounds regulating insect metamorphosis and development (IGR, e.g. juvenoids and their synthetic analogues) (Czech patent application PV 1680-95). Those compounds are toxic both to the attracted males, and also to those females which copulate with the contaminated males and subsequently pass the toxic principle on to their eggs.

The synthetic preparation of pheromones with conjugated carbon-carbon double bond system can be performed in several ways. One of them, providing us with limited possibility to control stereochemical purity of the prepared pheromones which is essential for the proper biological activity, is the Witting reaction of either α,β-unsaturated aldehydes with ylides prepared from alkyl triarylphosphonium salts or complementary with α,β-unsaturated ylides and aldehydes described in (Bestman, H.J., Suss, J. and Vostrowsky, O. Synthese konjugiert-ungesättiger Lepidopterenpheromone und Analoga. *Liebigs Ann*. *Chem*. **1981,** 2117-2138) who prepared besides others the (8E,10Z)-8,10-tetradecadien-1-ols derivatives. ZZ isomers, especially (11Z,13Z)-11,13-hexadecadiyn-1-ol, were prepared from easy available diynes using simultaneous reduction of both triple bonds with dicyclohexylborane (UP Patent 4,228,093). EZ, ZE and ZZ isomers can be prepared from E- or Z-enines using the same reduction procedure as above (Descoins C., Lettere, M., Linstrumelle, C., Michelot, D. and Ratovelomanana, V. Stereoselective Syntheses of (E)-7, (Z)-9 Dodecadien-1-yl Acetate. *Synthetic Commun.* 14, 761-773 (1984). The required enines are obtainable with easy from acetylenes coupled with the corresponding E- or Z-vinyl halides in the presence of catalytic amounts of palladium (0) complexes (Descoins C., Lettere, M., Linstrumelle, C., Michelot, D. and Ratovelomanana, V. Stereoselective Syntheses of (E)-7, (Z)-9 Dodecadien-1-yl Acetate. *Synthetic Commun*. 14, 761-773 (1984)). However, the most general strategy represents coupling of vinyl halides with vinyl organometallics (Koutek, B., Streinz, L., Romanuk, M. Synthesis of Insect Sex Pheromones. A Review of the Literature 1990-1998 *Collect. Czech. Chem. Commun*. 63, 899-954 (1998)). Alternatively, using stereoselective opening of silytepoxides with vinyl organocuprates assisted with Lewis acids and subsequent selective elimination of formed hydoxysilosones might be the most general method of preparation of all geometric isomers of conjugated sex pheromones (Svato 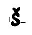 , A. and 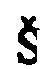 aman, D. Efficient stereoselective synthesis of all geometrical isomers of heptadeca-11,13-dienes. *Coll. Czech. Chem. Commun. 62,* 1457-1467 (1997)). In Ando, T., Koike, M., Uchiyama,M., and Kuroko, H. Lepidopterous Sex Attractants with a Conjugated Diene System. Agric. Biol. Chem. 51, 2691-2694 (1987) which relates to an overview of field tests with conjugated diene systems are disclosed tetradeca-8,10-dienals useful as lepidopterus sex attractant. There is also indicated that 8,10 dienes might be characteristic components of the Gracillariidae pheromones. This statement is based on tests with various (8E,10E)-8,10-tetradecadienes. Nevertheless no 8,10-tetradecadienes have been identified from female moths.

### Disclosure of Invention

Existing methods to control the leafminer *C. ohridella* are presently unsatisfactory, ecologically unsound (insecticide spraying in inhabited areas), or time consuming and not cost-effective (racking of leaves). This invention, based on the discovery of a sex pheromone and its method of preparation, eliminates these drawbacks, being based on the modification of chemical communication between the sexes by use of an identified and synthesized sex pheromone, with the aim of attracting and trapping males, or confusing them and thus interrupting the reproductive cycle of the leafminer. The subject of the present invention is the isomerically pure (*E*8,*Z*10)-tetradeca-8,10-dienal of the formula VI 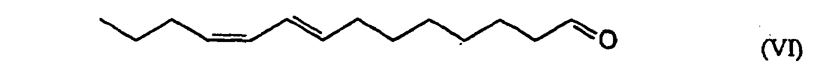 and the method of synthesizing thereof characterized in that the iodide of the formula VII 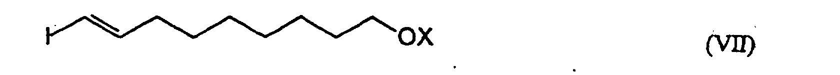 where X represents a protection group X as *tert*-butyl, 1-tetrahydropyranyl under an inert atmosphere of preferably nitrogen or argon, which is treated with a ca 2-5 fold excess of 1-pentyne in the presence of 1-5 equivalents of an amine preferably piperidine, 1-butylamine, a Pd(0) catalyst preferably tetrakis(trifenylfosphine)-palladium(0) in an amount of 0.5-20 mol % and a cuprous salt preferably cuprous iodide in 1-3 equivalents in an organic aprotic solvent, preferably selected from the group of benzene, toluene, and tetrahydrofuran, in a temperature range of 10-100 °C for 0.1 - 20 hr to obtain the compound of the formula VIII 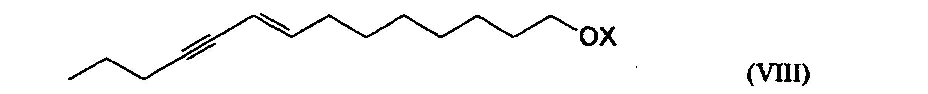 which is treated with dialkylborane preferably dicyclohexylborane in 1-3 equivalents for 0.1 - 5 hrs and the boron compound formed is hydrolyzed with 1-10 equivalents of an acid preferably acetic or propionic acid for 1-40 hrs at 10-80 °C and the protected dienole of formula IX obtained is 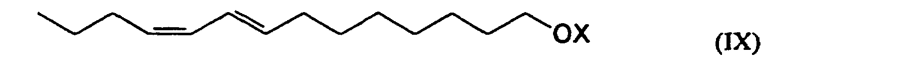 processed with acid preferably a mixture of ether/acetanhydride/ferric(III) chloride) at 10-40 °C for 1-50 hrs and then with 1-3 equivalents of potassium hydroxide dissolved in aqueous methanol at 20-50 °C for 1-24 hrs to provide the compound of formula X 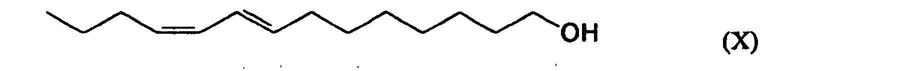 which is finally oxidized, preferably with Cr(VI) compounds selected from pyridinium chlorochromate or pyridinium dichromate in an appropriate solvent such as benzene, chloroform, or dichloromethane at 10-50 °C for 0.1-20 hrs and the resultant compound of formula VI is purified using column chromatography, preferably in a silica gel column and the synthetic pheromone is stored under an inert atmosphere, advantageously under nitrogen or argon, at 0-78 °C either as a neat substance or in the form of solutions in organic solvents, preferably in hexane or benzene.

The present invention further involves a method of using a compound of the general formula VI as a specific sex attractant for leafminers of the Gracillariidae (Lepidoptera) genus and a method of population monitoring, of mass trapping, of confusion and of contamination with biocontrol agents of males of the genus Gracillariidae in devices comprising compounds of the general formula I along with a mechanical restraining member, chemical or biological agents.

Further there is involved a method of using a compound of the general formula VI as a specific sex attractant for horse chestnut leafminer males (*Cameraria ohridella* Deschka et Dimié 1985, Lepidoptera: Gracillariidae) and a method of population monitoring, of mass trapping, of confusion and of contamination with biocontrol agents of horse chestnut leafminer males in devices comprising compounds described by the general formula VI.

The devices for carrying out the method of population monitoring, of mass trapping, of confusion and of contamination with biocontrol agents of males of the genus Gracillariidae comprising compounds of the general formula VI along with mechanical restraining member, chemical or biological agents and a pheromone dispenser. The pheromone dispenser being characterized in that the compound described by general formula VI is directly, in a solution, as a mixture with antioxidants and/or stabilizers or in a form of appropriate pro-pheromone derivatives applied on a suitable support selected from the group of natural or synthetic rubber polymers, cellulose glass fibers or metal films.

Devices for the contamination of males according to the present invention can contain, beside the attractant described by the general formula VI, insecticides, a biocontrol agent, or compounds regulating insect development (IGR -Insect Growth Regulators), in which the attracted males are killed or agents spread into leafminer populations.

The methods of preparation of the sex pheromone, under the terms of this invention, involve the use of easily available starting 1-alkenyliodides [e.g. Svat 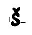 A., 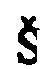 aman, D.: *Coll*. *Czech. Chem. Commun.* **62,** 1457-1467(1997)] with a suitable protection group (e.g. Greene, T.W.: *Protective groups in organic synthesis,* Wiley 1981). The iodide is coupled with an 1-alkyne in the presence of a catalytic amount of catalyst [Pd(0)]X₄ and an oxidative agent (typically a cuprous salt) in a basic solvent (amine) under an inert atmosphere to provide enynes [e.g. Descoins, C., Lethere, M., Linstrumelle, G., Michelot, D. Ratovelomanana V. *Syn. Commun.* 14, 761 (1984)]. The enyne can be stereospecifically reduced to a diene by dialkylboranes with subsequent hydrogenolysis of the alkenylboranes thus obtained [e.g. Brown, H.C., Mandal, A.K., Kulkarni, S.U. *J. Org. Chem.* 42, 1392 (1977)]. The protection group can be cleaved off in several ways (e.g. Greene, T.W.: *Protective groups in organic synthesis,* Wiley 1981), however, mild conditions are preferred to limit any possible isomerisation of the conjugated diene. Thus prepared alcohol is oxidized to an aldehyde by the action of various oxidation reagents, preferentially with pyridinium chlorochromate and pyridinium dichromate in an aprotic solvent [Corey, E.J., Suggs, J.W.: *Tetrahedron Let.* 31, 2647 (1975)]. The synthetic sex pheromone obtained is further purified in a silica gel column. Given the sensitivity of both the synthetic intermediates and the final product to oxidation and/or isomerisation of the double bonds, they should be kept under an inert atmosphere and at a low temperature (-20°C). The synthetic route as described is extremely favorable, despite its multi-step design, as high isomeric purity and chemical yields are easily obtainable and require no special qualification, unusual reagents or complicated production apparatus.

The biological activity of the title compound was proved by wind tunnel bioassays and field tests. The compound described by formula VI is then used for the preparation of lures or dispensers which are further employed in monitoring and trapping devices e.g. Czech patent No.: 257503 from 20.2.1990 (Czech patent app. No. PV 419-85), and in traps to enable the contamination of attracted males with insect killing agents, see Czech patent ..... (Patent application No. PV 1680-95).

The subject of the invention is supported by the examples of preparation and use described herein, however they do not limit it in any sense.

### Examples

### Example 1

### (E)-14-(tert-butoxy)tetradec-6-en-4-yne [VIII]

Tetrakis(triphenylphosphine)palladium (0.60 g, 0.5 mmol) was added at 20 °C to a solution of iodide 4 ((E)-9-(tert-butoxy)-1-iodonon-1-ene) (3.24 g, 10.0 mmol) in benzene (25 ml). The mixture was stirred for an additional 60 min. A solution of pent-1-yne (1.66 g, 15.0 mmol) in anhydrous butylamine (7.34 g, 100 mmol) and copper iodide (0.37 g, 2.0 mmol) was added. After 2 h at room temperature, the mixture was diluted with ether (150 ml) and subsequently poured into saturated aqueous ammonium chloride (100 ml). The organic layer was washed with 20% aqueous ammonia (3 × 100 ml), brine (2 × 100 ml), water (2 × 100 ml), and dried over anhydrous MgSO₄. Removal of the solvents *in vacuo* and purification of the residue by PMPLC (0.5 - 1.5% of ethyl acetate in hexane) gave 2.38 g (90%) of enyne **5**. ¹H NMR (CDCl₃): 0.98 t (3 H, *J*= 7.5, C**H**₃CH₂-); 1.18 s (9 H, 3 × C**H**₃); 1.25-1.39 m (8 H, 4 × **CH**₂); 1.50 m (2 H, CH₃C**H**₂-); 1.52 m (2 H, -C**H**₂CH₂OR); 2.07 dq (2 H, *J* = 1.5, 3 × 7.1, -CH=CHC**H**₂-); 2.26 dt (2 H, *J =* 1.2, 2 × 1.7, -C**H**₂C≡C-); 3.32 t (2 H, *J* = 6.7, -C**H**₂OR); 5.45 m (1 H, *J* = 4 × 1.7, 15.8, -CH=C**H**-); 6.03 m (1 H, *J* = 4 × 1.7, 15.8, -C**H**=CH-).

### Example 2

### (4Z,6E)-14-(tert-Butoxy)tetradeca-4,6-diene (6)

A dicyclohexylborane suspension was prepared in THF (30 ml) from borane-dimethyl sulfide complex (10 M, 0.97 ml) and cyclohexene (1.59 g, 19.4 mmol). The white suspension obtained was treated at 0 °C with a solution of enyne **5** (2.33 g, 8.8 mmol) in THF (10 ml). The mixture was warmed to room temperature, and stirred for 4 h. The vinylborane formed was hydrolyzed with glacial acetic acid (10 ml) at 20 °C for 12 h, the reaction mixture was neutralized with NaOH (20%, 20 ml), and carefully treated with aqueous H₂O₂ (30%, 10 ml). The product was extracted with hexane (4 × 75 ml). Chromatography (PMPLC) of the hexane yielded protected dienol **6** (1.61 g, 69%) of 97.8% isomeric purity (GC). ¹H NMR (CDCl₃): 0.92 t (3 H, *J* = 7.4, C**H**₃CH₂-); 1.18 s (9 H, 3 × C**H**₃); 1.24-1.36 m (8 H, 4 × C**H**₂); 1.40 m (2 H, *J=* 5 × 7.4, CH₃C**H**₂-); 1.51 m (2 H, -C**H**₂CH₂OR); 2.09 bdq (2 H, *J* = 1.5, 3 × 7.5, -CH=CHC**H**₂-); 2.14 dq (2 H, *J* = 1.5, 3 × 7.5, -C**H**₂CH=CH-); 3.32 t (2 H, *J* = 6.7, -C**H**₂OR); 5.3 0 bdt (1 H, *J =* 2 × 7.5, 10.8, -C**H**=CH-CH=CH-); 5.65 bdt (1 H, *J =* 3 × 7.3, 15.0, -CH=CH-CH=C**H**-); 5.95 dtt (1 H, *J* = 0.8, 2 × 1.5,2 × 10.9, -CH=C**H**-CH=CH-); 6.29 ddq (1 H, *J* = 3 × 1.5, 11.0, 15.0, -CH=CH-C**H**=CH-).

### Example 3

### (8E,10Z)-Tetradeca-8,10-dien-1-ol (7)

Acetic anhydride (2 ml), and then anhydrous FeCl₃ (65 mg, 0.40 mmol) were added to a solution of protected dienol **6** (1.07 g; 4.0 mmol) in ether (20 ml). The dark brown solution was stirred for 20 h at room temperature. A saturated aqueous solution of Na₂HPO₄ (15 ml) was added, and the mixture was stirred for 2 h. The solid FePO₄ was filtered off, and the aqueous layer was extracted with ether (3 × 40 ml). The collected organic phases were dried over anhydrous MgSO₄, and then concentrated. The red oily residue (1.03 g) was dissolved in methanol (10 ml) and an aqueous solution of NaOH (0.60 g in 4 ml of water) was added. The mixture was stirred at 20 °C for 16 h, poured into water (75 ml), and extracted with hexane - ether (3 : 2, 4 × 25 ml). The combined extracts were dried (anhydrous K₂CO₃), evaporated, and the dark residue (0.98 g) was purified by PMPLC. The chromatography (10% ethyl acetate in hexane) gave 0.69 g (82%) of *E*,*Z*-dienol 7. ¹H NMR (CDCl₃): 0.92 t (3 H, *J* = 7.4, CH₃CH₂-); 1.25-1.39 m (8 H, 4 × C**H**₂); 1.41 m (2 H, *J =* 5 × 7.4, CH₃C**H**₂-); 1.57 m (2 H, -C**H**₂CH₂OR); 2.09 bdq (2 H, *J* = 1.5, 3 × 7.1, -CH=CHC**H**₂-); 2.14 dq (2 H, *J* = 1.6, 3 × 7.5, -C**H**₂CH=CH-); 3.65 t (2 H, *J* = 6.6, -C**H**₂OH); 5.31 dtt (1 H, *J =* 2 × 0.7, 2 × 7.5, 10.8, -C**H**=CH-CH=C**H**-); 5.65 dtt (1 H, *J =* 2 × 0.8, 2 × 7.1, 15.1, -CH=CH-CH=C**H**-); 5.96 dtt (1 H, *J* = 0.9, 2 × 1.6, 2 × 10.9, -CH=C**H**-CH=CH-); 6.30 ddq (1 H, *J* = 3 × 1.5, 11.0, 15.1, -=CH-C**H**=CH-).

### Example 4

### (8E,10Z)- Tetradeca-8,10-dienal (1a)

Dienol 7 (210 mg, 1 mmol) was injected into a stirred suspension of pyridinium chlorochromate (PCC; 258 mg, 1.2 mmol) and anhydrous sodium acetate (20 mg) in dichloromethane (2 ml). The mixture was stirred for 90 min at room temperature, then poured into 50 ml of ether and filtered through a combined layer of neutral alumina / charcoal / Celite. A subsequent evaporation of the solvents and PMPLC (0.3% triethylamine in benzene - hexane / 1 : 1) afforded 151 mg (72%) of the pure (96%, GC) target compound, dienal **1a**. ¹H NMR (CDCl₃): 0.92 t (3 H, *J* = 7.4, C**H**₃CH₂-); 1.27-1.38 m (6 H, 3 × C**H**₂); 1.40 m (2 H, *J*= 5 × 7.4, CH₃C**H**₂-); 1.63 m (2 H, -C**H**₂CH₂CHO); 2.09 bdq (2 H, *J* = 1.5, 3 × 7.1, -CH=CHC**H**₂-); 2.14 dq (2 H, *J* = 1.6, 3 × 7.5, -C**H**₂CH=CH-); 2.42 dt (2 H, J = 2 × 6.5, -C**H**₂CHO); 5.31 dtt (1 H, *J =* 2 × 0.7, 2 × 7.5, 10.8, -C**H**=CH-CH=CH-); 5.65 dtt (1 H, *J =* 2 × 0.8, 2 × 7.0, 15.1, -CH=CH-CH=C**H**-), 5.95 dtt (1 H, *J* = 0.9, 2 × 1.6, 2 × 10.9, -CH=C**H**-CH=CH-); 6.30 ddq (1 H, *J* = 3 × 1.5, 11.0, 15.1, -CH=CH-C**H**=CH-); 9.76 t (1 H, *J* = 1.8, -C**H**O). EI-MS, *m*/*z* (rel. %): 39 (11), 41 (33), 54 (23), 55 (27), 67 (100), 68 (18), 79 (33), 80 (13), 81 (50), 82 (21), 91 (16), 93 (14), 95 (26), 96 (18), 98 (19), 109 (14), 208 (9, M^{+.}).

### Example 5

### Pheromone dispenser

Compound **VI** is dissolved in hexane of benzene to form a 10 mg/l solution, 10 microliters of which is applied onto rubber septum (that is 100 ng/dispenser). The solvent is left to evaporate and the pheromone dispenser thus prepared is stored in a glass vial at -20 °C.

### Example 6

### Usage of compound VI in a field test

The pheromone dispenser (example 5) and a sticky insert covered with Tanglefoot glue is introduced into Delta trap (25 × 10 cm) and this monitoring device is suspended ca 2.5-3 m above ground near horse chestnut trees and the number of males caught is periodically recorded. Some results are presented in the Table 1.

**Table 1.**

| Catches of horse chestnut males in traps according to example 6 | |
|---|---|
| **Lure** | **males caught/day** |
| control | 0 |
| 3 caged horse chestnut leafminer females | 65 |
| compound of structure **VI** | 50 |

## Claims

1. Isomerically pure (*E*8,*Z*10)-Tetradeca-8,10-dienal of the formula VI

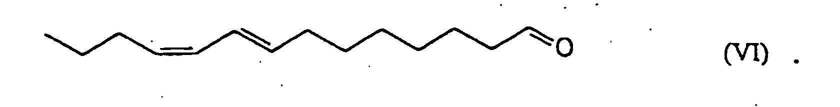

2. Method for the preparation of the compound of formula VI according to claim 1, **characterized by** the following steps:
(A) Reaction of an iodide compound of general formula VII, 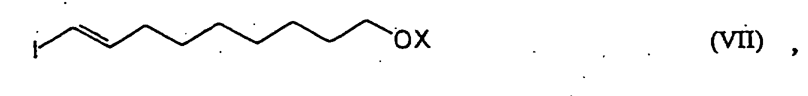 wherein X represents a protection group selected from *tert*-butyl and 1-tetrahydropyranyl,
with 1-pentyne in a 2-5 fold excess under an inert atmosphere in the presence of 1 to 5 equivalents of an amine, a Pd(0) catalyst in an amount of 0.5 to 20 mol-% and 1 to 3 equivalents of a cuprous salt in an organic aprotic solvent at 10 to 100 °C for 0.1 to 20 h
to obtain the compound of the formula VIII, 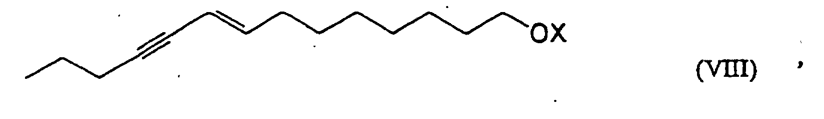
(B) reduction of the compound of formula VIII with a dialkylborane in an amount of 1 to 3 equivalents for 0.1 to 5 h,
(C) hydrolysis of the boron compound formed in step B with 1 to 10 equivalents of an acid for 1 to 40 h at 10 to 80 °C to obtain a protected dienole of formula IX, 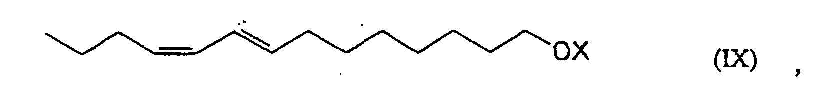
(D) deprotection of the compound of formula IX by treatment with an acid at 10 to 40 °C for 1 to 50 h and then with 1 to 3 equivalents of potassium hydroxide dissolved in aqueous methanol at 20 to 50 °C for 1 to 24 h to obtain the compound of formula X, 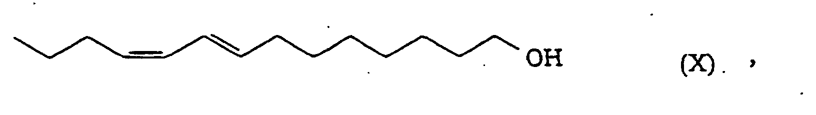
(E) final oxidation of the compound of formula X with a Cr(VI) compound in an appropriate solvent at 10 to 50 °C for 0.1 to 20 h to obtain the compound of formula VI
and
(F) purification of the compound of formula VI by column chromatography.

3. Method according to claim 2, **characterized in that** the synthetic pheromone of formula VI obtained after step (F) is stored under an inert atmosphere, preferably under nitrogen or argon, at 0 to -78 °C either as neat substance or in the form of solutions in organic solvents, preferably in hexane or benzene.

4. Method according to claim 2, **characterized by** one or more of the following measures:
- In step (A), nitrogen or argon is used as inert gas;
- in step (A), piperidine or 1-butylamine is used as amine;
- in step (A), tetrakis(triphenylphosphine)palladium(0) is used as Pd(0) catalyst;
- in step (A), cuprous iodide is used as cuprous salt;
- in step (A), benzene, toluene or tetrahydro-furan is used as organic aprotic solvent;
- in step (B), dicyclohexylborane is used as dialkylborane;
- in step (C), acetic acid or propionic acid is used as acid for hydrolysis;
- in step (D), an ether/acetanhydride/iron(III)chloride mixture is used as acid;
- in step (E), pyridinium chlorochromate or pyridinium dichromate is used as Cr(VI) compound;
- in step (E), benezene, chloroform or dichloromethane is used as solvent;
- in step (F), a silica gel column is used.

5. Use of the compound of formula VI according to claim 1 as specific sex attractant for leafminers of the genus of Gracillariidae (Lepidoptera).

6. Use of the compound of formula VI according to claim 1 as a specific sex attractant for horse chestnut leafminer males (*Cameraria ohridella*, Gracillariidae, Lepidoptera).

7. Method of population monitoring, of mass trapping, of confusion and of contamination with biocontrol agents of males of the genus of Gracillariidae in devices comprising compounds of the general formula VI according to claim 1, along with a mechanical restraining member, chemical or biological agents.

8. Method of population monitoring, of mass trapping, of confusion and of contamination with biocontrol agents of horse chestnut leafminer males in devices comprising compounds of formula VI according to claim 1.

9. Devices comprising compounds of formula VI according to claim 1 for performing the methods of claims 7 and 8, comprising a pheromone dispenser.

10. Pheromone dispenser according to claim 9, **characterized in that** the compound of formula VI is directly, in a solution, as a mixture with antioxidants and/or stabilizers or in a form of appropriate pro-pheromone derivates applied on a suitable support selected from natural and synthetic rubber polymers, cellulose glass fibers and metal films.

11. Devices for the contamination of males of the genus of Gracillariidae, **characterized in that** the devices contain, besides the attractant according to claim 1, insecticides, a biocontrol agent, or compounds regulating insect development, in which the attracted males are killed or agents spread into the leafminer populations.

## Patentansprüche

1. Isomerenreines (E8, Z10)-Tetradeca-8,10-dienal der Formel VI

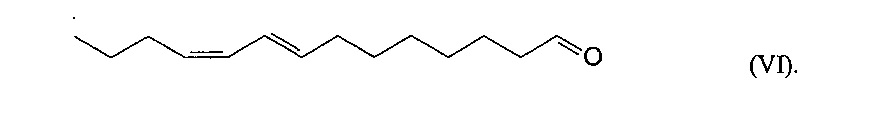

2. Verfahren zur Herstellung der Verbindung der Formel VI nach Anspruch 1, **gekennzeichnet durch** folgende Schritte:
(A) Umsetzung einer Jodid-Verbindung gemäß der allgemeinen Formel VII, 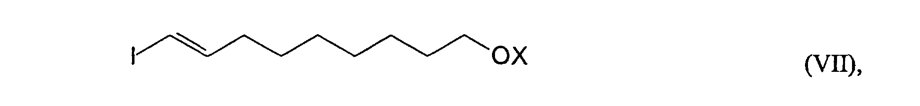 wobei X eine Schutzgruppe darstellt, die unter tert-Butyl und 1-Tetrahydropyranyl ausgewählt ist,
mit 1-Pentin in einem 2,5fachen Überschuss in einer inerten Atmosphäre in Gegenwart von 1 bis 5 Äquivalenten eines Amins, eines Pd(0)-Katalysators in einer Menge von 0,5 bis 20 mol % und von 1 bis 3 Äquivalenten eines Kupfer(I)-Salzes in einem organischen aprotischen Lösungsmittel bei 10 bis 100 °C während 0,1 bis 20 h
unter Erhalt der Verbindung der Formel VIII, 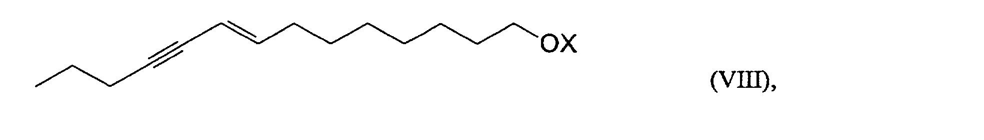
(B) Reduktion der Verbindung der Formel VIII mit einem Dialkylboran in einer Menge von 1 bis 3 Äquivalenten während 0,1 bis 5 h,
(C ) Hydrolyse der in Schritt B gebildeten Borverbindung mit 1 bis 10 Äquivalenten einer Säure während 1 bis 40 h bei 10 bis 80 °C unter Erhalt eines geschützten Dienols der Formel IX, 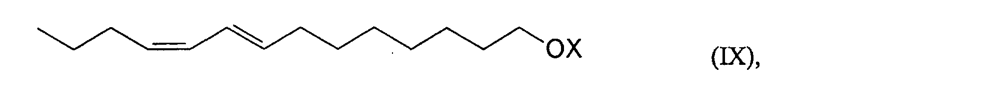
(D) Entfernen der Schutzgruppe der Verbindung der Formel IX **durch** Behandlung mit einer Säure bei 10 bis 40 °C während 1 bis 50 h, und danach mit 1 bis 3 Äquivalenten Kaliumhydroxid, gelöst in wässerigem Methanol bei 20 bis 50 °C während 1 bis 24 h unter Erhalt der Verbindung der Formel X, 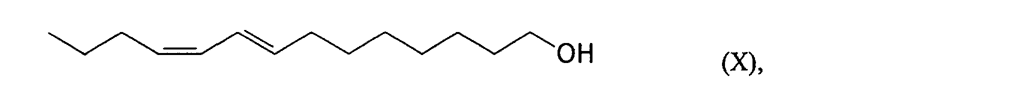
(E) abschließende Oxidation der Verbindung der Formel X mit einer Cr(VI)-Verbindung in einem geeigneten Lösungsmittel bei 10 bis 50 °C während 0,1 bis 20 h unter Erhalt der Verbindung der Formel VI
und
(F) Reinigung der Verbindung der Formel VI **durch** Säulenchromatographie.

3. Verfahren nach Anspruch 2, **dadurch gekennzeichnet, dass** das nach Schritt (F) erhaltene synthetische Pheromon der Formel VI unter einer inerten Atmosphäre, vorzugsweise unter Stickstoff oder Argon, bei 0 bis -78 °C entweder als reine Substanz oder in Form von Lösungen in organischen Lösungsmitteln, bevorzugt in Hexan oder Benzol aufbewahrt wird.

4. Verfahren nach Anspruch 2, **gekennzeichnet durch** eine oder mehrere der folgenden Maßnahmen:
- in Schritt (A) wird als Inertgas Stickstoff oder Argon verwendet;
- in Schritt (A) wird als Amin Piperidin oder 1-Butylamin verwendet;
- in Schritt (A) wird als Pd(0)-Katalysator Tetrakis(triphenylphosphin)palladium(0) verwendet;
- in Schritt (A) wird als Kupfer (I)Salz Kupfer(I)iodid verwendet;
- in Schritt (A) wird als organisches aprotisches Lösungsmittel Benzol, Toluol oder Tetrahydrofuran verwendet;
- in Schritt (B) wird als Dialkylboran Dicyclohexylboran verwendet;
- in Schritt (C ) wird als Säure für die Hydrolyse Essigsäure oder Propionsäure verwendet;
- in Schritt (D) wird als Säure ein Gemisch von Ether/Acetanhydrid/Eisen(III)-chlorid-Gemisch verwendet;
- in Schritt (E) wird als Cr(VI)-Verbindung Pyridinium-chlorchromat oder Pyridiniumdichromat verwendet;
- in Schritt (E) wird als Lösungsmittel Benzol, Chloroform oder Dichlormethan verwendet;
- in Schritt (F) wird eine Kieselsäuregel-Säule verwendet.

5. Verwendung der Verbindung der Formel VI nach Anspruch 1 als spezifischen Sexuallockstoff für Miniermotten der Gattung Gracillariidae (Lepidoptera).

6. Verwendung der Verbindung der Formel VI nach Anspruch 1 als spezifischen Sexuallockstoff für die Männchen der Rosskastanien-Miniermotte (*Cameraria ohridella*, Gracilladiidae, Lepidoptera).

7. Verfahren zur Populationsüberwachung, zum Massenfang, zur Konfusion und zur Kontaminierung durch Biokontrollmittel von Männchen der Gattung Gracillariidae in Vorrichtungen, die Verbindungen der allgemeinen Formel VI nach Anspruch 1, zusammen mit einem mechanischen Rückhalteelement, chemischen oder biologischen Mitteln, enthalten.

8. Verfahren zur Populationsüberwachung, zum Massenfang, zur Konfusion und zur Kontaminierung durch Biokontrollmittel von Männchen der Rosskastanien-Miniermotte in Vorrichtungen, die Verbindungen der Formel VI nach Anspruch 1 enthalten.

9. Vorrichtungen, die Verbindungen der Formel VI nach Anspruch 1 und einen Pheromondispenser enthalten, zur Durchführung der Verfahren der Ansprüche 7 und 8.

10. Pheromondispenser nach Anspruch 9, **dadurch gekennzeichnet, dass** die Verbindung gemäß Formel VI direkt, in einer Lösung, als Gemisch mit Antioxidantien und/oder Stabilisatoren oder in Form von entsprechenden Pro-Pheromonderivaten auf eine geeignete Unterlage aufgebracht ist, die unter natürlichen und synthetischen Kautschuk-Polymeren, Cellulose, Glasfasern und Metallfilmen, ausgewählt ist.

11. Vorrichtungen zur Kontaminierung von Männchen der Gattung Gracillariidae, **dadurch gekennzeichnet, dass** die Vorrichtungen neben dem Lockstoff nach Anspruch 1 Insektizide, Biokontrollmittel oder Verbindungen zur Regulierung der Insektenentwicklung enthalten, in welchen die angelockten Männchen getötet werden oder Mittel in den Minimiermotten-Populationen verbreitet werden.

## Revendications

1. (E8, Z10)-Tétradeca-8,10-diénal isomériquement pur selon la formule VI

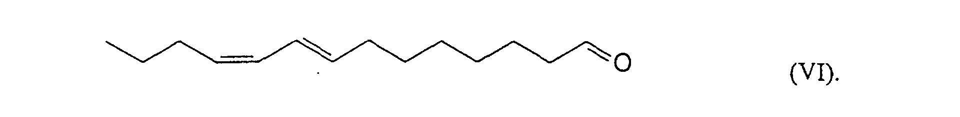

2. Procédé pour la préparation du composé de la formule VI selon la revendication 1, **caractérise par** les étapes suivantes:
(A) réaction d'un composé iodée selon la formule générale VII, 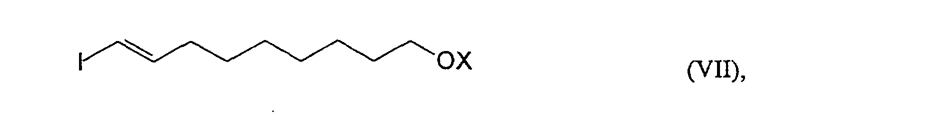 où X représente un groupe de protection choisi parmi le *tert*-butyl et le 1-tetrahydropyranyl,
avec 1-pentine dans un excès 2-5 fois plus élevé sous une atmosphère inerte en présence de 1 à 5 équivalents d'un amine, d'un catalyseur Pd(0) dans une quantité de 0,5 à 20 mol-% et de 1 à 3 équivalents d'un sel cuivreux dans un solvant organique aprotique à une température de 10 à 100° C pendant 0,1 à 20 h,
pour obtenir le composé de la formule VIII, 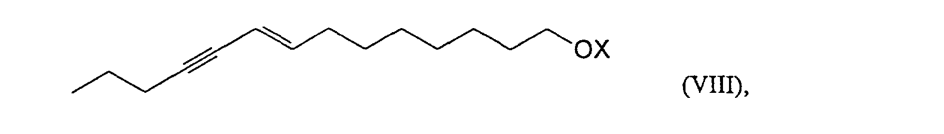
(B) réduction du composé de la formule VIII par un dialkyle borane dans une quantité de 1 à 3 équivalents pendant 0,1 à 5 h,
(C) hydrolyse du composé de bore formé dans l'étape B avec 1 à 10 équivalents d'un acide pendant 1 à 40 h à une température de 10 à 80° C pour obtenir un diénol protégé de la formule IX, 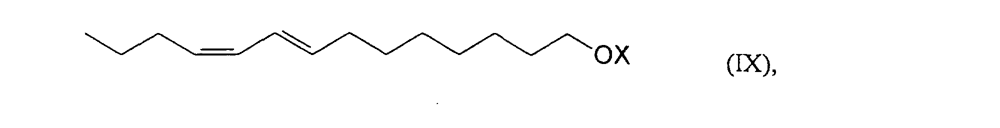
(D)élimination du groupe de protection du composé de la formule IX par traitement avec un acide à une température de 10 à 40° C pendant 1 à 50 h, et puis avec 1 à 3 équivalents d'hydroxyde de potassium dissous dans un méthanol aqueux à une température de 20 à 50° C pendant 1 à 24 h pour obtenir le composé de la formule X, 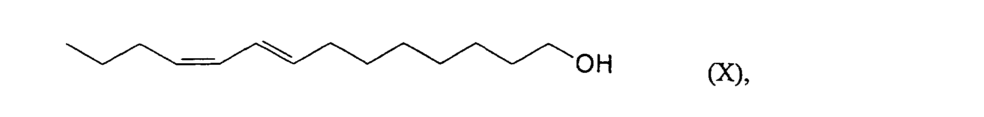
(E) oxydation finale du composé de la formule X avec un composé de Cr(VI) dans un solvant approprié à une température de 10 à 50° C pendant 0,1 à 20h pour obtenir le composé de la formule VI
et
(F) purification du composé de la formule VI par chromatographie sur colonne échangeuse.

3. Procédé selon la revendication 2, **caractérisé en ce que** la phéromone synthétique de la formule VI obtenue après l'étape (F) est conservée sous atmosphère inerte, de préférence une atmosphère d'azote ou d'argon, à une température de 0 à 78° C, soit sous forme de substance pure, soit en forme de solutions dans des solvants organiques, de préférence dans de l'hexane ou du benzène.

4. Procédé selon la revendication 2, **caractérisé par** l'une ou plusieurs des mesures suivantes:
- dans l'étape (A), de l'azote ou de l'argon est utilisé en tant que gaz inerte;
- dans l'étape (A), de la pipéridine ou de la 1-butylamine est utilisée en tant qu'amine;
- dans l'étape (A), du tetrakis(triphenylphosphine)palladium(0) est utilisé en tant que catalyseur Pd(0);
- dans l'étape (A), du iodure de cuivre(I) est utilisé en tant que sel cuivreux ;
- dans l'étape (A), du benzène, du toluène ou du tetrahydrofurane est utilisé en tant que solvant aprotique organique ;
- dans l'étape (B), du dicyclohexylborane est utilisé en tant que dialkyleborane ;
- dans l'étape (C), de l'acide acétique ou de l'acide propionique est utilisé en tant qu'acide pour l'hydrolyse ;
- dans l'étape (D), un mélange d'éther/acétanhydride/chlorure de fer(III) est utilisé en tant qu'acide ;
- dans l'étape (E), du chlorochromate de pyridinium ou du dichromate de pyridinium est utilisé en tant que composé de Cr(VI) ;
- dans l'étape (E), du benzène, du chloroforme ou du dichlorométhane est utilisé en tant que solvant ;
- dans l'étape (F), une colonne de gel de silice est utilisée.

5. Utilisation du composé de la formule VI selon la revendication 1 comme un appât sexuel spécifique pour la mineuse du marronnier de la famille des Gracillariidae (Lepidoptera).

6. Utilisation du composé de la formule VI selon la revendication 1 comme un appât sexuel spécifique pour les mâles de la mineuse du marronnier d'Inde (*Cameraria ohridella*, Gracillariidae, Lepidoptera).

7. Méthode de monitorage de la population, de rattrapage en masse, de confusion et de contamination par des agents de contrôle biologique, des mâles de la famille de Gracillariidae dans les dispositifs comportant des composés de la formule générale VI selon la revendication 1, en combinaison avec un élément de retenue mécanique, des agents chimiques ou biologiques.

8. Méthode de monitorage de la population, de rattrapage en masse, de confusion et de contamination par les agents de contrôle biologique, des mâles de la mineuse du marronnier d'Inde dans des dispositifs comportant des composés de la formule VI selon la revendication 1.

9. Dispositifs comportant des composés de la formule VI selon la revendication 1 pour la réalisation les procédés des revendications 7 et 8, comprenant un distributeur de phéromone.

10. Distributeur de phéromone selon la revendication 9, **caractérisé en ce que** le composé de la formule VI est appliqué directement, dans une solution, sous forme de mélange avec des antioxydants et/ou des stabilisants ou sous forme des dérivés de pro-phéromones correspondants, sur un support approprié choisi parmi les polymères de caoutchouc naturel et synthétique, les fibres en verre, la cellulose et les films métalliques.

11. Dispositifs pour la contamination des mâles de la famille des Gracillariidae, **caractérisés en ce que** les dispositifs comportent, outre l'appât selon la revendication 1, des insecticides, des agents de contrôle biologique ou des composés réglant le développement des insectes, dans lesquels les mâles attirés sont tués ou des agents sont distribués dans les populations de la mineuse du marronnier.
